# EUROPEAN PATENT SPECIFICATION

(11) **EP 2 800 400 B1**
(45) Date of publication and mention of the grant of the patent: **04.07.2018**
(21) Application number: 12862613.2
(22) Date of filing: 22.11.2012
(51) Int. Cl.: H01L 41/083, H01L 41/18, A61B 17/32, B06B 1/06, A61B 8/00

(54) **ULTRASOUND TRANSDUCER DEVICE AND ULTRASOUND MEDICAL APPARATUS**
ULTRASCHALLWANDLERVORRICHTUNG UND MEDIZINISCHES ULTRASCHALLGERÄT
DISPOSITIF TRANSDUCTEUR ULTRASONORE ET APPAREIL MÉDICAL À ULTRASONS

(30) Priority: 26.12.2011 JP 2011283670
(43) Date of publication of application: 05.11.2014
(73) Proprietor: Olympus Corporation, Tokyo 192-8507 (JP)
(72) Inventor: ITO, Hiroshi, Tokyo 192-8507 (JP)
(74) Representative: Winter, Brandl, Fürniss, Hübner, Röss, Kaiser, Polte - Partnerschaft mbB
(86) International application number: PCT/JP2012/080315
(87) International publication number: WO 2013/099482

(56) References cited:
- JP-A- 2004 080 193
- JP-A- 2007 195 389
- JP-A- 2011 139 295
- US-A1- 2008 294 087
- MATSUNAMI G ET AL: "Multilayered LiNbO3 actuator for XY-stage using a shear piezoelectric effect", SENSORS AND ACTUATORS A, ELSEVIER SEQUOIA S.A., LAUSANNE, CH, vol. 144, no. 2, 15 June 2008 (2008-06-15) , pages 337-340, XP022664312, ISSN: 0924-4247, DOI: 10.1016/J.SNA.2008.02.006 [retrieved on 2008-03-10]
- KAWAMATA ET AL: "Non-hysteresis and perfect linear piezoelectric performance of a multilayered lithium niobate actuator", SENSORS AND ACTUATORS A: PHYSICAL, ELSEVIER BV, NL, vol. 135, no. 2, 4 April 2007 (2007-04-04) , pages 782-786, XP022016245, ISSN: 0924-4247, DOI: 10.1016/J.SNA.2006.08.025

## Description

### Technical Field

The present invention relates to an ultrasound transducer device that excites ultrasound vibrations and an ultrasound medical apparatus including the ultrasound transducer device.

### Background Art

In recent years, ultrasound medical apparatuses including ultrasound transducers are known. Examples of the ultrasound medical apparatuses include an ultrasound diagnostic apparatus that images an internal state of a living body and an ultrasound scalpel for coagulation and dissection in a surgery. In these apparatuses, piezoelectric materials are used for the ultrasound transducer that generates ultrasound vibrations from an electric signal, and piezoelectric ceramics typified by PZT (lead zirconate titanate) and piezoelectric single crystals are used therefor. Moreover, the ultrasound transducer may be formed by stacking such piezoelectric materials in a plurality of layers, for the purpose of a reduction in impedance and an increase in output power.

For example, a technique disclosed in Japanese Patent Application Laid-Open Publication No. 2001-102650 is known as an ultrasound transducer formed by stacking piezoelectric single crystals. The conventional transducer is a stacked piezoelectric single-crystal element formed by: putting two or more single crystals ((1-x)Pb(B1, B2)O3-xPbTiO3 (when x = 0-0.55, B1 = Mg, Zn, Ni, Sc, In, Yb, Lu, B2 = Nb, Ta)) having an AB03 type perovskite structure on top of each other, using a metal material for bonding; bonding the single crystals to each other by heating; and then polarizing the piezoelectric single-crystal elements.

A further device of the prior art is known from Go Matsunami et al., "Multilayered LiNbO3 actuator for XY-stage using a shear piezoelectric effect", Sensors and Actuators A, vol. 144, p. 337-340, (2008).

The single-crystal material used for the conventional transducer is a uniaxial material, and is polarized in a stacking direction of the piezoelectric elements. Hence, the stacking direction is an axis of rotational symmetry, and deformation in a direction perpendicular to the stacking direction, that is, deformation in a plane of the transducer is the same irrespective of orientation.

However, for some piezoelectric single-crystal materials, the stacking direction is not an axis of rotational symmetry, a piezoelectric constant is different depending on orientation, and a strain pattern is different. A Langevin type transducer is described as an example. In the Langevin type transducer, at least two or more piezoelectric single-crystal materials whose stacking direction is not an axis of rotational symmetry are stacked on each other, and polarization components in the stacking direction are alternately inverted. In the Langevin type transducer configured as described above, because the piezoelectric single-crystal materials are stacked on each other such that front surfaces and rear surfaces of respective piezoelectric elements are alternately inverted, there is a problem that deformation in an in-plane direction perpendicular (orthogonal) to the stacking direction, that is, deformation in an in-plane direction of the piezoelectric transducer cannot be made coincident between adjacent piezoelectric transducers in all directions. Further, in the conventional Langevin type transducer, because strain in the in-plane direction is not coincident between adjacent piezoelectric transducers, deformation in the in-plane direction is hindered, and this influences deformation in the stacking direction, so that an excess stress acts on between layers in which deformation due to a same input voltage becomes smaller. Accordingly, the transducer becomes more liable to be broken, and is more likely to be damaged at the time of driving.

The present invention, which has been made in view of the above-mentioned circumstances, has an object to enable providing an ultrasound transducer device that can minimize a hindrance to deformation in an in-plane direction of a piezoelectric transducer and an excess stress acting on between adjacent transducers, can prevent damage at the time of driving, and can more efficiently obtain deformation in a transducer stacking direction, and an ultrasound medical apparatus using the ultrasound transducer device.

### Disclosure of Invention

### Means for Solving the Problem

An ultrasound transducer device according to the present invention is defined by claim 1. Moreover, an ultrasound medical apparatus according to an aspect of the present invention includes an ultrasound transducer device including a plurality of piezoelectric single-crystal plates that are stacked such that polarization components thereof are alternately inverted. The plurality of piezoelectric single-crystal plates are stacked such that directions thereof in which strain deformation in a direction orthogonal to a direction of voltage application from electrodes respectively interposed between the plurality of piezoelectric single-crystal plates becomes largest coincide with each other.

According to the present invention described above, it is possible to provide an ultrasound transducer device that can minimize a hindrance to deformation in an in-plane direction of a piezoelectric transducer and an excess stress acting on between adjacent transducers, can prevent damage at the time of driving, and can more efficiently obtain deformation in a piezoelectric single-crystal plates stacking direction, and an ultrasound medical apparatus using the ultrasound transducer device.

### Brief Description of the Drawings

Fig. 1 is a cross-sectional view illustrating an overall configuration of an ultrasound medical apparatus according to an aspect of the present invention;
Fig. 2 is a view illustrating an overall schematic configuration of a transducer unit according to the aspect of the present invention;
Fig. 3 is a perspective view illustrating a configuration of an ultrasound transducer according to the aspect of the present invention;
Fig. 4 is a partial cross-sectional view illustrating the configuration of the ultrasound transducer according to the aspect of the present invention;
Fig. 5 is a cross-sectional view illustrating a configuration of a stacked transducer according to the aspect of the present invention;
Fig. 6 is a perspective view illustrating a single-crystal wafer according to the aspect of the present invention;
Fig. 7 is a plan view illustrating the single-crystal wafer according to the aspect of the present invention, which is observed from a polished surface side;
Figs. 8 illustrate a strain pattern of each piezoelectric single-crystal plate according to the aspect of the present invention, Fig. 8(a) is a view illustrating a voltage application direction, Fig. 8(b) is a view illustrating perpendicular (orthogonal) strain, and Fig. 8(c) is a view illustrating shear strain;
Fig. 9 is a graph showing substrate in-plane direction dependencies of piezoelectric strain constants according to the aspect of the present invention;
Fig. 10 is a graph showing a substrate in-plane direction dependency of a piezoelectric strain constant obtained by adding the perpendicular strain and the shear strain, according to the aspect of the present invention;
Fig. 11 is a schematic view illustrating a relative relation of a wafer coordinate system of the stacked transducer according to the aspect of the present invention;
Fig. 12 is a plan view illustrating a first example of each piezoelectric single-crystal plate according to the aspect of the present invention and illustrating a direction in which deformation thereof becomes largest;
Fig. 13 is a plan view illustrating a second example of each piezoelectric single-crystal plate according to the aspect of the present invention and illustrating a direction in which deformation thereof becomes largest;
Fig. 14 is a plan view illustrating a third example of each piezoelectric single-crystal plate according to the aspect of the present invention and illustrating a direction in which deformation thereof becomes largest;
Fig. 15 is a plan view illustrating one surface of each piezoelectric single-crystal plate provided with an electrode on which a first index portion is formed, according to the aspect of the present invention; and
Fig. 16 is a plan view illustrating the other surface of each piezoelectric single-crystal plate provided with an electrode on which a second index portion is formed, according to the aspect of the present invention.

### Best Mode for Carrying Out the Invention

Hereinafter, the present invention is described with reference to the drawings. Note that, in the following description, because the drawings based on each embodiment are schematic, a relation in thickness and width among respective portions, a ratio in thickness of each portion, and the like are different from actual values thereof, and a portion in which a relation and a ratio of mutual dimensions are different among the drawings may be included.

### (Ultrasound Medical Apparatus)

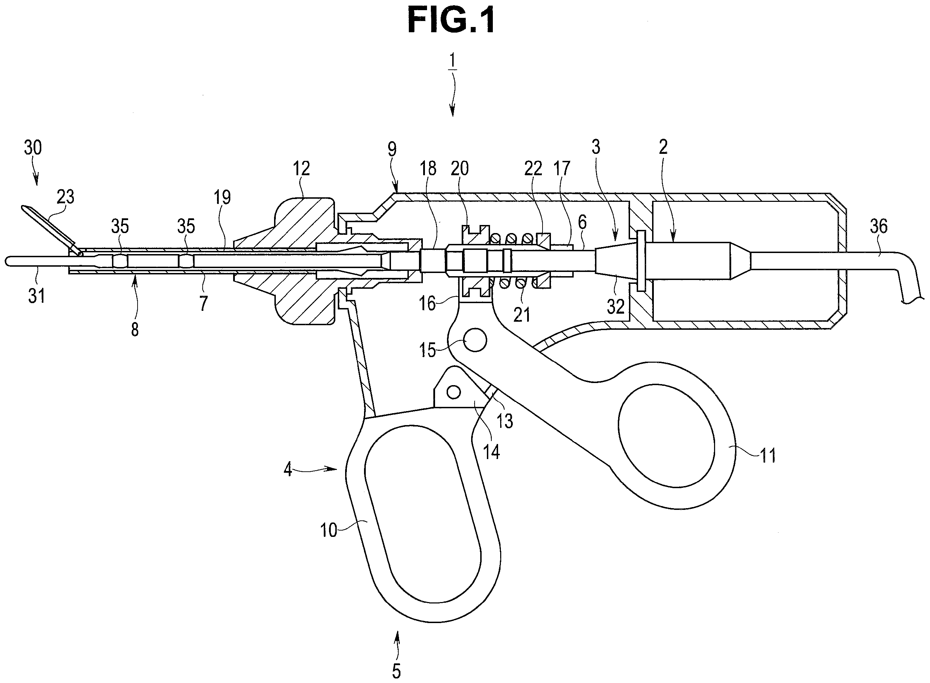

Fig. 1 is a cross-sectional view illustrating an overall configuration of an ultrasound medical apparatus according to the present embodiment.

An ultrasound medical apparatus 1 illustrated in Fig. 1 is mainly provided with: a transducer unit 3 including an ultrasound transducer 2 as an ultrasound device that generates ultrasound vibrations; and a handle unit 4 that treats an affected area using the ultrasound vibrations.

The handle unit 4 includes an operation portion 5, an insertion sheath portion 8 formed by an elongated cover tube 7, and a distal-end treatment portion 30. A proximal end portion of the insertion sheath portion 8 is attached to the operation portion 5 so as to be rotatable about an axis thereof. The distal-end treatment portion 30 is provided at a distal end of the insertion sheath portion 8. The operation portion 5 of the handle unit 4 includes an operation portion main body 9, a fixed handle 10, a movable handle 11, and a rotating knob 12. The operation portion main body 9 is formed integrally with the fixed handle 10.

A slit 13 through which the movable handle 11 is inserted is formed on a back side in a coupling part between the operation portion main body 9 and the fixed handle 10. An upper portion of the movable handle 11 is extended inside of the operation portion main body 9 through the slit 13. A handle stopper 14 is fixed to a lower end portion of the slit 13. The movable handle 11 is turnably attached to the operation portion main body 9 by means of a handle support shaft 15. Then, along with a turning motion of the movable handle 11 about the handle support shaft 15, the movable handle 11 is operated to be opened and closed with respect to the fixed handle 10.

A substantially U-shaped coupling arm 16 is provided in an upper end portion of the movable handle 11. Moreover, the insertion sheath portion 8 includes the cover tube 7 and an operation pipe 17 that is inserted through an inside of the cover tube 7 so as to be movable in an axis direction. A larger-diameter portion 18 having a diameter larger than that of a distal end portion of the cover tube 7 is formed in a proximal end portion of the cover tube 7. The rotating knob 12 is fitted around the larger-diameter portion 18.

A ring-shaped slider 20 is provided on an outer circumferential surface of an operation pipe 19 so as to be movable along the axis direction. A fixed ring 22 is arranged behind the slider 20 with the intermediation of a coil spring (elastic member) 21.

Further, a proximal end portion of a grasping portion 23 is turnably coupled to a distal end portion of the operation pipe 19 by means of an action pin. The grasping portion 23 constitutes a treatment portion of the ultrasound medical apparatus 1 together with a distal end portion 31 of a probe 6. Then, at the time of a moving motion of the operation pipe 19 in the axis direction, the grasping portion 23 is operated to be pushed and pulled in a front-back direction by means of the action pin. At this time, when the operation pipe 19 is operated to move toward an operator's hand side, the grasping portion 23 is turned about a fulcrum pin by means of the action pin. Consequently, the grasping portion 23 is turned in a direction (closing direction) in which the grasping portion 23 approaches the distal end portion 31 of the probe 6. At this time, a living tissue can be grasped between the grasping portion 23 of single swing type and the distal end portion 31 of the probe 6.

In such a state where the living tissue is grasped, electric power is supplied from an ultrasound power supply to the ultrasound transducer 2, and the ultrasound transducer 2 is vibrated. The resultant ultrasound vibrations are transmitted to the distal end portion 31 of the probe 6. Then, coagulation and dissection of the living tissue grasped between the grasping portion 23 and the distal end portion 31 of the probe 6 is performed using the ultrasound vibrations.

### (Transducer Unit)

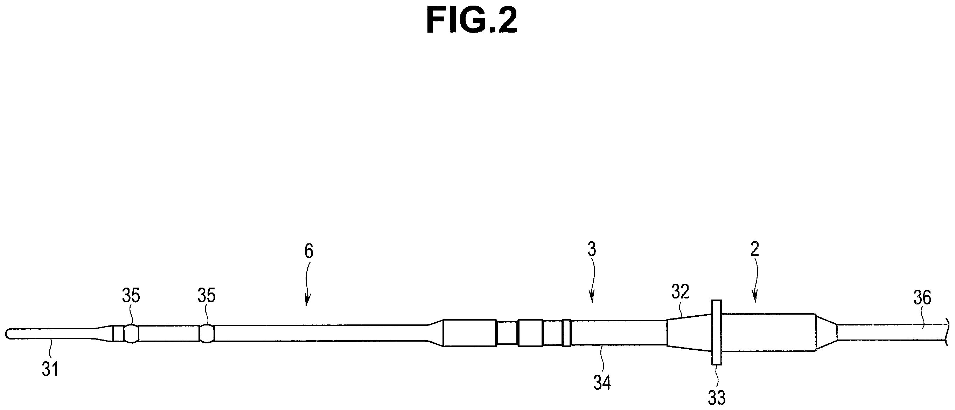

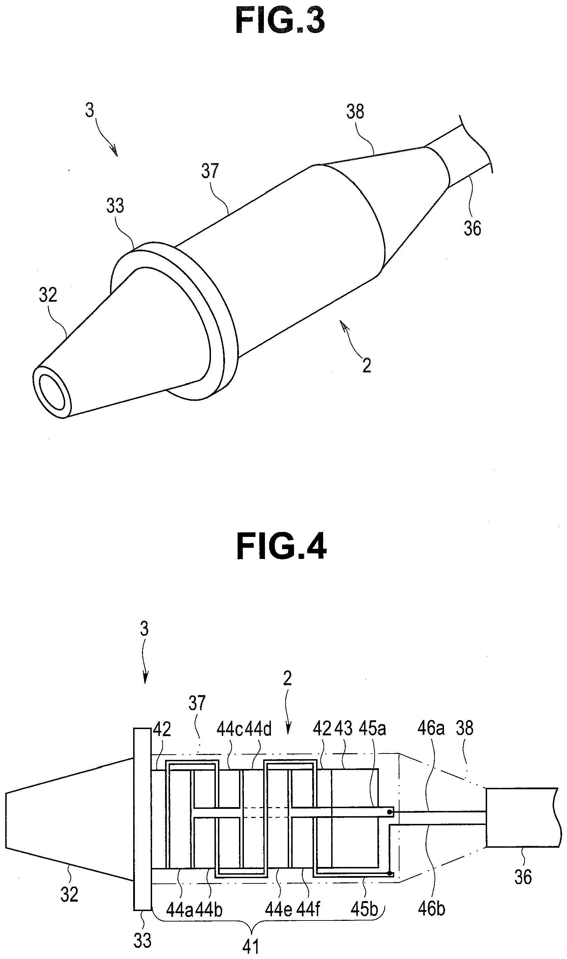

Here, the transducer unit 3 is described. Note that Fig. 2 is a view illustrating an overall schematic configuration of the transducer unit 3, and Fig. 3 is a perspective view illustrating an overall schematic configuration of the ultrasound transducer.

As illustrated in Fig. 2 and Fig. 3, the ultrasound transducer 2 and the probe 6, which is a rod-shaped vibration transmitting member that transmits ultrasound vibrations generated by the ultrasound transducer 2, are integrally incorporated in the transducer unit 3.

A horn 32 that amplifies an amplitude of the ultrasound vibration is provided continuously with the ultrasound transducer 2. The horn 32 is made of duralumin or a titanium alloy such as 64Ti. The horn 32 is formed in a conical shape having an outer diameter that becomes smaller toward a distal end side thereof, and an outward flange 33 is formed in a proximal-end outer circumferential portion of the horn 32.

The probe 6 includes a probe main body 34 made of a titanium alloy such as 64Ti. The ultrasound transducer 2 provided continuously with the horn 32 is arranged on a proximal end portion side of the probe main body 34. In this way, the transducer unit 3 in which the probe 6 and the ultrasound transducer 2 are integrated with each other is formed.

Then, the ultrasound vibrations generated by the ultrasound transducer 2 are amplified by the horn 32, and are then transmitted to the distal end portion 31 side of the probe 6. The treatment portion (to be described later) that treats a living tissue is formed in the distal end portion 31 of the probe 6.

Moreover, on an outer circumferential surface of the probe main body 34, two rubber linings 35 that are each formed into a ring shape using an elastic member are attached with a space therebetween at several vibration node positions in the middle of the axis direction. Then, the rubber linings 35 prevent the outer circumferential surface of the probe main body 34 and the operation pipe 19 to be described later from coming into contact with each other. That is, at the time of assembling of the insertion sheath portion 8, the probe 6 as a transducer-integrated probe is inserted into the operation pipe 19. At this time, the rubber linings 35 prevent the outer circumferential surface of the probe main body 34 and the operation pipe 19 from coming into contact with each other.

Note that the ultrasound transducer 2 is electrically connected to a power supply apparatus main body (not illustrated) that supplies current for generating ultrasound vibrations, via an electric cable 36. Electric power is supplied from the power supply apparatus main body to the ultrasound transducer 2 through wires in the electric cable 36, whereby the ultrasound transducer 2 is driven.

### (Ultrasound Transducer)

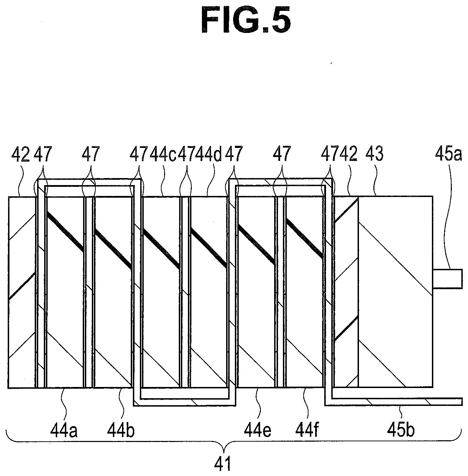

Here, the ultrasound transducer 2 of the transducer unit 3 is described below. Note that Fig. 4 is a partial cross-sectional view illustrating the configuration of the ultrasound transducer, and Fig. 5 is a cross-sectional view illustrating a configuration of a stacked transducer.

As illustrated in Fig. 3 and Fig. 4, the ultrasound transducer 2 as the ultrasound device of the transducer unit 3 includes: a cylindrical case body 37 joined to the horn 32; and a bend preventer 38 behind which the electric cable 36 extends, the bend preventer 38 being provided continuously with a proximal end of the case body 37, in order from the distal end.

A stacked transducer 41 is arranged in the case body 37. Insulating plates 42 are respectively provided on a distal end side and a proximal end side of the stacked transducer 41. A plurality of (here, six) piezoelectric single-crystal plates 44a to 44f are stacked between the insulating plate 42 on the distal end side that is fixed to a proximal end surface of the horn 32 and the insulating plate 42 on the proximal end side that is joined to and provided continuously with a front side of a back mass 43. The piezoelectric single-crystal plates 44a to 44f are stacked such that polarization components in a stacking direction are alternately inverted between adjacent plates.

Moreover, in order to enable voltage application to the respective piezoelectric single-crystal plates 44a to 44f, a bendable positive electrode plate 45a and a bendable negative electrode plate 45b made of copper foil are alternately sandwiched between the respective piezoelectric single-crystal plates 44a to 44f, and are extended behind the stacked transducer 41. Note that each of the electrode plates 45a, 45b is connected to a same polarization surface of each of the piezoelectric single-crystal plates 44a to 44f.

The electrode plates 45a, 45b are respectively connected to wires 46a, 46b arranged in the electric cable 36. Then, the respective electrode plates 45a, 45b apply voltage to the piezoelectric single-crystal plates 44a to 44f, and ultrasonically vibrate the stacked transducer 41 in the stacking direction of the piezoelectric single-crystal plates 44a to 44f, due to a piezoelectric effect.

Moreover, as illustrated in Fig. 5, the insulating plates 42, the back mass 43, the piezoelectric single-crystal plates 44a to 44f, and the respective electrode plates 45a, 45b are bonded to and integrated with one another using a joining material 47. Examples of the joining material 47 include organic materials such as conductive adhesives and metal materials such as solders. Moreover, the stacked transducer 41 is configured as a bolt-clamped Langevin transducer, and the horn 32 and the back mass 43 are fastened to each other using bolts, whereby the horn 32, the insulating plates 42, the back mass 43, the piezoelectric single-crystal plates 44a to 44f, and the respective electrode plates 45a, 45b may be integrated with one another.

### (Piezoelectric Single-Crystal Plate)

Hereinafter, the piezoelectric single-crystal plates 44a to 44f used in the present embodiment are described below. Note that description is given of a case where the piezoelectric single-crystal plates 44a to 44f here are made of LiNb03 (lithium niobate) that is a lead-free single-crystal material not containing lead (Pb) and where a 36° Y-cut substrate suitable to obtain vibrations in a wafer thickness direction is used for each of the piezoelectric single-crystal plates 44a to 44f.

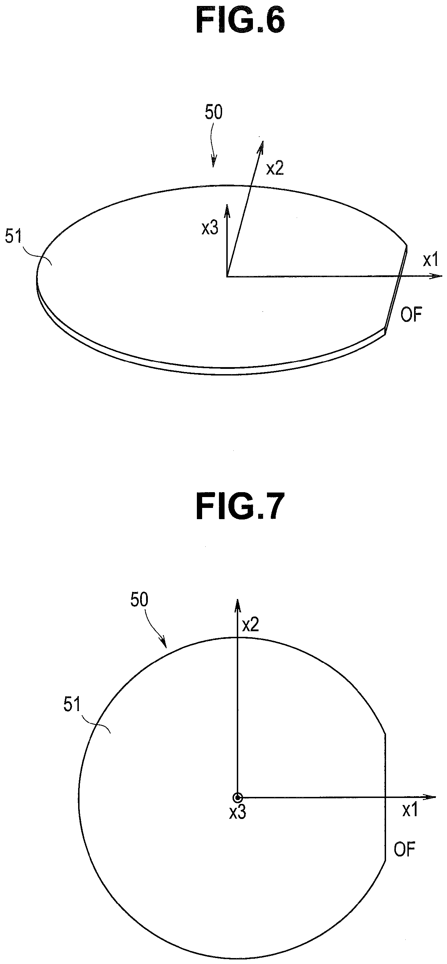

Note that Fig. 6 is a perspective view illustrating a single-crystal wafer, and Fig. 7 is a plan view illustrating the single-crystal wafer, which is observed from a polished surface side.

A LiNb03 single-crystal wafer 50 illustrated in Fig. 6 and Fig. 7 is processed into such a wafer shape that has a particular orientation with respect to crystal axes (X, Y, Z), in order to obtain desired characteristics depending on an intended use. For example, although a wafer called 128° Y-cut is used for a SAW (surface acoustic wave) device, in the piezoelectric single-crystal plates 44a to 44f of the present embodiment, vibrations in the stacking direction are obtained by stacking LiNb03 piezoelectric single crystals, and hence the 36° Y-cut substrate that makes a piezoelectric constant in the stacking direction larger is suitable.

A direction of the LiNb03 single-crystal wafer 50 to each crystal axis is defined by an Euler angle. In a coordinate system (x1, x2, x3) on the wafer 50, a direction perpendicular (orthogonal) to a polished surface 51 of the wafer 50 is defined as an x3 axis, an OF (orientation flat) direction from a center of the wafer 50 is defined as an x1 axis, and an x2 direction is selected such that the x1 axis, an x2 axis, and the x3 axis form a right-handed orthogonal coordinate system.

The crystal axes (X, Y, Z) of the LiNbO3 single crystal used for each of the piezoelectric single-crystal plates 44a to 44f and the coordinate system (x1, x2, x3) on the wafer 50 are associated with each other by Euler angles (φ, ψ, θ). The polished surface 51 plane of the wafer 50 is determined by the Euler angles φ, ψ, and the OF (orientation flat) direction, that is, a direction of the x3 axis is determined by the Euler angle θ. Each of the piezoelectric single-crystal plates 44a to 44f here is processed into a rectangular or discoid chip through dicing or machining of a LiNb03 36° Y-cut substrate having such a particular orientation that the Euler angles with respect to the crystal axes (X, Y, Z) are (180°, 54°, 180°).

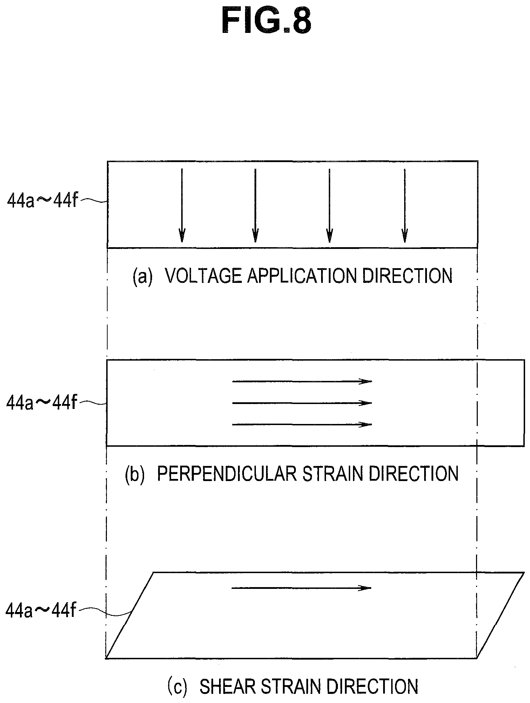

Figs. 8 illustrate deformation in a direction perpendicular (orthogonal) to a voltage application direction in a case where voltage is applied in a thickness direction of each of the piezoelectric single-crystal plates 44a to 44f. Note that Figs. 8 illustrate a strain pattern of each piezoelectric single-crystal plate, Fig. 8(a) is a view illustrating the voltage application direction, Fig. 8(b) is a view illustrating perpendicular (orthogonal) strain, and Fig. 8(c) is a view illustrating shear strain. As illustrated in Figs. 8, the strain pattern of each of the piezoelectric single-crystal plates 44a to 44f includes two types of strain, that is, the perpendicular (orthogonal) strain illustrated in Fig. 8(b) and the shear strain illustrated in Fig. 8(c), with respect to the voltage application direction illustrated in Fig. 8(a).

For the perpendicular strain in the piezoelectric single-crystal plates 44a to 44f, the piezoelectric single-crystal plates 44a to 44f wholly expand and contract in the direction orthogonal to the voltage application direction. In contrast, for the shear strain in the piezoelectric single-crystal plates 44a to 44f, a voltage application surface is displaced in the direction orthogonal to the voltage application direction, and a cross-section of each of the piezoelectric single-crystal plates 44a to 44f obliquely strains.

A magnitude of strain when voltage is applied to the piezoelectric single-crystal plates 44a to 44f is expressed by a piezoelectric strain constant d. Assuming that the voltage application direction is the three axis directions of the coordinate system, the perpendicular strain is expressed by d31, d32, and the shear strain is expressed by d35, d34. In a case of a piezoelectric single crystal as in the present embodiment, the piezoelectric constant is different in a transducer plane depending on a direction, due to crystal anisotropy.

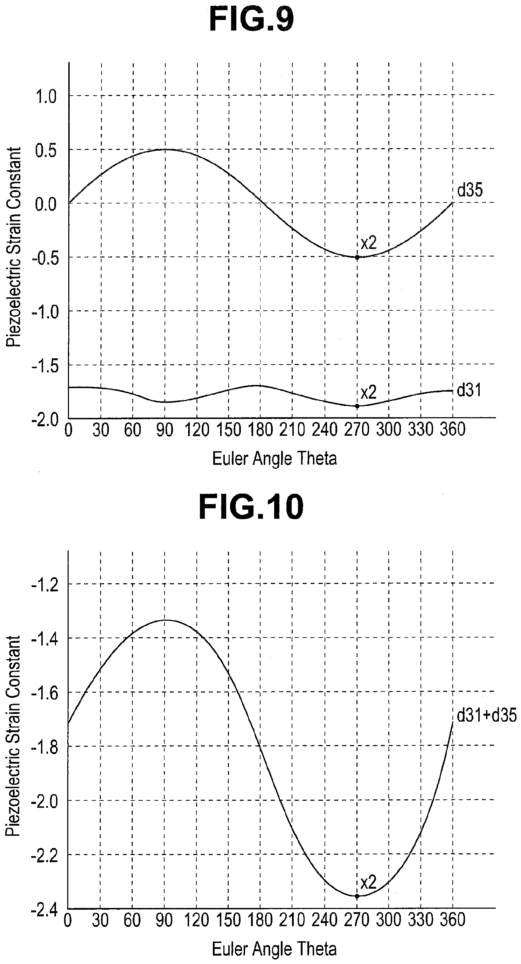

Here, 36° Y-cut substrate in-plane direction dependencies of the piezoelectric strain constants d31, d35 are described. Note that Fig. 9 is a graph showing substrate in-plane direction dependencies of piezoelectric strain constants, and Fig. 10 is a graph showing a substrate in-plane direction dependency of a piezoelectric strain constant obtained by adding the perpendicular strain and the shear strain.

An x axis of the graph shown in Fig. 9 is the Euler angle θ, and represents a direction in the wafer 50 plane. Moreover, Fig. 10 shows a piezoelectric strain constant d31 + d35. Note that an absolute value of each value represents a magnitude of strain. As is apparent from these graphs, both the piezoelectric strain constants d31, d35 become largest, and the shear strain becomes largest, in a direction of the Euler angle θ = 270 degrees, that is, in the direction of the x2 axis in the wafer coordinate system of Fig. 6 and Fig. 7.

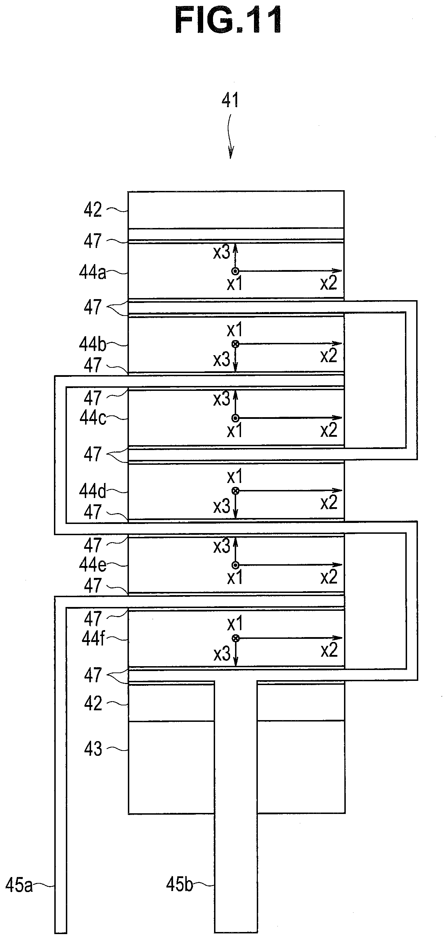

Accordingly, as illustrated in Fig. 11, the stacked transducer 41 of the ultrasound transducer 2 of the present invention is configured as the Langevin transducer in the following manner. That is, front sides and rear sides of the respective piezoelectric single-crystal plates 44a to 44f are alternately stacked such that the x2 axes thereof in the coordinate system on the wafer 50 coincide with each other, in terms of a relative relation of the wafer coordinate system. Note that Fig. 11 is a schematic view illustrating the relative relation of the wafer coordinate system of the stacked transducer.

As a result, in the ultrasound transducer 2, a difference in in-plane deformation becomes smallest between adjacent ones of the piezoelectric single-crystal plates 44a to 44f of the stacked transducer 41. Accordingly, a hindrance to deformation in the in-plane direction can be reduced, damage at the time of driving can be prevented, and vibrations in the stacking direction can be efficiently obtained.

Further, because the ultrasound transducer 2 is formed using the piezoelectric single-crystal plates 44a to 44f based on the LiNb03 36° Y-cut substrates each made of a lead-free single-crystal material, the ultrasound transducer 2 can have a configuration suitable for non-lead environmental protection that has been desired in recent years. Moreover, the piezoelectric single-crystal plates 44a to 44f are not limited to the lithium niobate single-crystal material, and, for example, a lithium tantalate piezoelectric single crystal can also be used therefor as long as front sides and rear sides of the piezoelectric single-crystal plates 44a to 44f are alternately stacked such that the axes thereof in the wafer coordinate system along which the shear strain becomes largest coincide with each other.

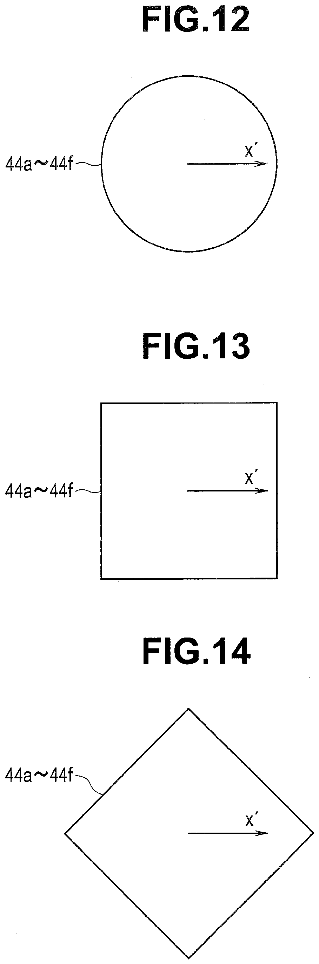

Note that, as illustrated in Fig. 12 to Fig. 14, an outer shape of each of the piezoelectric single-crystal plates 44a to 44f is processed into a circular or rectangular shape such that a direction (in the drawings, an x' direction) in which deformation in a direction orthogonal to the stacking direction (in-plane deformation of a transducer single plate) becomes largest is an axis of line symmetry. If the outer shape is processed into such a shape, excited ultrasound vibrations are made stable, and an outer circumferential shape of the stacked transducer 41 is made even because the piezoelectric single-crystal plates 44a to 44f are stacked such that the x' axis directions thereof coincide with each other. In particular, in a case where the outer shape of the transducer is processed into the rectangular shape, when the piezoelectric single-crystal plates 44a to 44f are stacked such that the x' axis directions thereof coincide with each other and that polarization components thereof in the stacking direction are alternately inverted, the outer shape of the stacked body becomes a continuous plane, and hence the stacked transducer 41 is more easily manufactured. Note that Fig. 11 to Fig. 14 are plan views each illustrating a direction in which deformation in each piezoelectric single-crystal plate becomes largest.

Here, if the wafer 50 is processed into chips, that is, the piezoelectric single-crystal plates 44a to 44f, the following problem arises: a relation between the coordinate system on the wafer 50 and orientations of the chips cannot be recognized by appearance. In view of this, a step of forming electrodes in a wafer shape is provided, the electrodes are patterned, and marks that enable identification of an axis of symmetry and front and rear surfaces are formed on the axis of symmetry on the front and rear surfaces, respectively, whereby recognition of an orientation of a transducer can be facilitated. Normally, in a case where a plurality of transducers are integrated with each other by solders or the like, an electrode forming step is necessary. Hence, in such a case, desired marks can be created without the need to add an extra process.

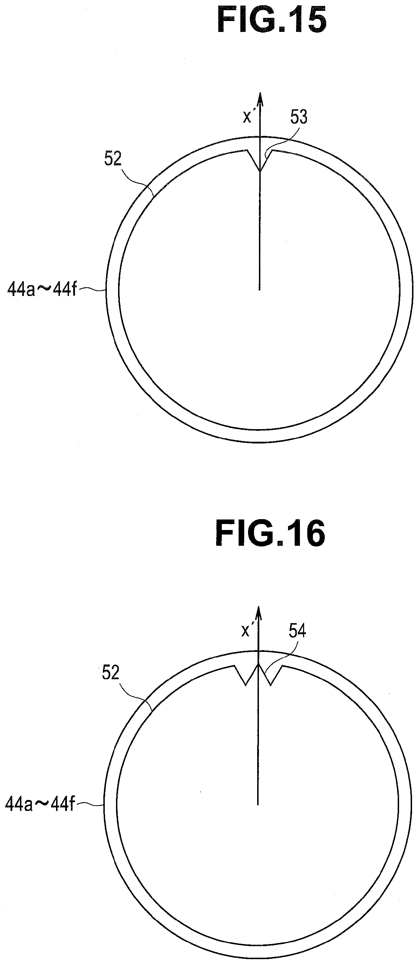

For example, as illustrated in Fig. 15 and Fig. 16, each of the piezoelectric single-crystal plates 44a to 44f has one surface provided with an electrode 52 on which a first index portion 53 is formed and the other surface provided with an electrode 52 on which a second index portion 54 is formed. Note that Fig. 15 is a plan view illustrating the one surface of each piezoelectric single-crystal plate provided with the electrode on which the first index portion is formed, and Fig. 16 is a plan view illustrating the other surface of each piezoelectric single-crystal plate provided with the electrode on which the second index portion is formed.

The electrodes 52 are respectively formed on both the surfaces of each of the piezoelectric single-crystal plates 44a to 44f through metal film formation and patterning. The metal film formation is performed using vapor deposition, sputtering, plating, and the like that are generally adopted, and the pattering is performed using photolithography, etching, and the like.

As the indexes, the first index portion 53 of one notch is formed as an electrode pattern on one main surface on the axis of symmetry, and the second index portion 54 of two notches having a shape different from that of the first index portion 53 is formed as an electrode pattern on the opposite surface.

Then, the piezoelectric single-crystal plates 44a to 44f are stacked by bringing the electrodes 52 into surface contact with each other such that the first index portions 53 match to each other and the second index portions 54 match to each other, whereby the stacked transducer 41 of the ultrasound transducer 2, which is stacked such that the polarization components in the stacking direction are alternately inverted and that the directions (x' directions) in which deformation in the direction orthogonal to the stacking direction (in-plane deformation of the transducer single plate) becomes largest coincide with each other, can be easily created. Note that shapes of the index portions 53, 54 may be any shapes as long as the front and rear surfaces of the piezoelectric single-crystal plates 44a to 44f and a position of the axis of symmetry can be identified.

The invention described in the above-mentioned embodiment is not limited to the embodiment and modifications thereof, but defined by the claims.

## Claims

1. An ultrasound Langevin transducer device (2) comprising a plurality of piezoelectric single-crystal plates (44a-44f) that are stacked such that polarization components thereof are alternately inverted, wherein
the plurality of piezoelectric single-crystal plates are stacked such that directions thereof in which strain deformation based on a sum of perpendicular strain and shear strain in a direction orthogonal to a direction of voltage application from electrodes (47) respectively interposed between the plurality of piezoelectric single-crystal plates becomes largest coincide with each other, the perpendicular strain being defined as strain in which the piezoelectric single-crystal plates wholly expand and contract orthogonal to the direction of voltage application, the shear strain being defined as strain in which a cross-section of the piezoelectric single-crystal plates obliquely strains in the direction orthogonal to the direction of voltage application.

2. The ultrasound transducer device according to claim 1, wherein the plurality of piezoelectric single-crystal plates are each made of a lead-free material.

3. The ultrasound transducer device according to claim 2, wherein the plurality of piezoelectric single-crystal plates are each made of a lithium niobate or lithium tantalate piezoelectric single crystal.

4. The ultrasound transducer device according to any one of claim 1 to claim 3, wherein the plurality of piezoelectric single-crystal plates are each bilaterally symmetric with respect to a direction in which strain deformation in a direction orthogonal to a stacking direction becomes largest, as an axis of symmetry.

5. The ultrasound transducer device according to claim 4, wherein the plurality of piezoelectric single-crystal plates each include index portions on the axis of symmetry on front and rear surfaces thereof, respectively, the index portions enabling identification of the direction in which the strain deformation becomes largest and the front and rear surfaces.

6. The ultrasound transducer device according to claim 5, wherein
the electrodes are respectively pattern-formed on the front and rear surfaces of the plurality of piezoelectric single-crystal plates, and
parts of the electrodes are respectively formed as the index portions into such different shapes that enable identification of the direction in which the strain deformation becomes largest and the front and rear surfaces.

7. An ultrasound medical apparatus comprising the ultrasound transducer device according to any one of claim 1 to claim 6.

## Patentansprüche

1. Langevin-Ultraschallwandlervorrichtung (2) mit mehreren piezoelektrischen Einkristallplatten (44a-44f), die so gestapelt sind, dass Polarisierungskomponenten davon abwechselnd invertiert sind, wobei
die mehreren piezoelektrischen Einkristallplatten so gestapelt sind, dass Richtungen davon, in denen eine Verformung auf der Grundlage einer Summe aus einer Normalspannung und einer Scherspannung in eine Richtung senkrecht zu einer Richtung, in der von Elektroden (47), die jeweils zwischen den mehreren piezoelektrischen Einkristallplatten angeordnet sind, am größten wird, miteinander zusammenfallen, wobei die Normalspannung als eine Spannung definiert ist, bei der sich die piezoelektrischen Einkristallplatten insgesamt ausdehnen und senkrecht zu der Richtung, in der die Spannung angelegt ist, zusammenziehen, wobei die Scherverformung als eine Verformung definiert ist, bei der sich ein Querschnitt der piezoelektrischen Einkristallplatten in der Richtung senkrecht zu der Richtung, in der die Spannung angelegt ist, schräg verformt.

2. Langevin-Ultraschallwandlervorrichtung nach Anspruch 1, wobei jede der mehreren piezoelektrischen Einkristallplatten aus einem bleifreien Material hergestellt ist.

3. Langevin-Ultraschallwandlervorrichtung nach Anspruch 2, wobei jede der mehreren piezoelektrischen Einkristallplatten aus einem piezoelektrischen Lithiumniobat- oder Lithiumtantalat-Einkristall hergestellt ist.

4. Langevin-Ultraschallwandlervorrichtung nach einem der Ansprüche 1 bis 3, wobei jeder der mehreren piezoelektrischen Einkristallplatten bilateral symmetrisch bezüglich einer Richtung als einer Symmetrieachse ist, in der eine Verformung in einer Richtung senkrecht zu einer Stapelrichtung am größten wird.

5. Langevin-Ultraschallwandlervorrichtung nach Anspruch 4, wobei jede der mehreren piezoelektrischen Einkristallplatten Indexabschnitte auf der Symmetrieachse auf einer vorderen bzw. einer hinteren Oberfläche davon umfassen, wobei die Indexabschnitte eine Identifizierung der Richtung, in der die Verformung am größten wird, und der vorderen und der hinteren Oberfläche ermöglichen.

6. Langevin-Ultraschallwandlervorrichtung nach Anspruch 5, wobei
die Elektroden auf der vorderen bzw. der hinteren Oberfläche der mehreren piezoelektrischen Einkristallplatten in einem Muster gebildet sind, und
Teile der Elektroden als die jeweiligen Indexabschnitte in solch unterschiedlichen Formen gebildet sind, dass eine Identifizierung der Richtung, in der die Verformung am größten wird, und der vorderen und der hinteren Oberfläche ermöglicht ist.

7. Medizinische Ultraschallvorrichtung, die die Langevin-Ultraschallwandlervorrichtung gemäß einem der Ansprüche 1 bis 6 umfasst.

## Revendications

1. Dispositif transducteur ultrasonore de Langevin (2) comprenant une pluralité de plaques monocristallines piézoélectriques (44a à 44f) qui sont empilées de telle sorte que des composants de polarisation en sont inversés en alternance, dans lequel
la pluralité de plaques monocristallines piézoélectriques sont empilées de telle sorte que leurs directions dans lesquelles une déformation d'effort basée sur une somme d'un effort perpendiculaire et d'un effort de cisaillement dans une direction orthogonale à une direction d'application de tension à partir d'électrodes (47) respectivement interposées entre la pluralité de plaques monocristallines piézoélectriques se fait la plus grande coïncident les unes avec les autres, l'effort perpendiculaire étant défini comme un effort dans lequel les plaques monocristallines piézoélectriques se dilatent et se contractent complètement orthogonalement à la direction d'application de tension, l'effort de cisaillement étant défini comme un effort dans lequel une section transversale des plaques monocristallines piézoélectriques subit un effort en oblique dans la direction orthogonale à la direction d'application de tension.

2. Dispositif transducteur ultrasonore selon la revendication 1, dans lequel la pluralité de plaques monocristallines piézoélectriques sont chacune constituées d'un matériau exempt de plomb.

3. Dispositif transducteur ultrasonore selon la revendication 2, dans lequel la pluralité de plaques monocristallines piézoélectriques sont chacune constituées d'un monocristal piézoélectrique de niobate de lithium ou de tantalate de lithium.

4. Dispositif transducteur ultrasonore selon l'une quelconque des revendications 1 à 3, dans lequel la pluralité de plaques monocristallines piézoélectriques sont chacune symétriques bilatéralement par rapport à une direction dans laquelle une déformation d'effort dans une direction orthogonale à une direction d'empilement se fait la plus grande, en tant qu'axe de symétrie.

5. Dispositif transducteur ultrasonore selon la revendication 4, dans lequel la pluralité de plaques monocristallines piézoélectriques incluent chacune des portions d'indice sur l'axe de symétrie sur des surfaces avant et arrière de celles-ci, respectivement, les portions d'indice permettant une identification de la direction dans laquelle la déformation d'effort se fait la plus grande et les surfaces avant et arrière.

6. Dispositif transducteur ultrasonore selon la revendication 5, dans lequel
les électrodes sont respectivement formées en motif sur les surfaces avant et arrière de la pluralité de plaques monocristallines piézoélectriques, et
des parties des électrodes sont respectivement formées comme les parties d'indice en des formes différentes telles qu'elles permettent une identification de la direction dans laquelle la déformation d'effort se fait la plus grande et les surfaces avant et arrière.

7. Appareil médical ultrasonore comprenant le dispositif transducteur ultrasonore selon l'une quelconque des revendications 1 à 6.
